# EUROPEAN PATENT APPLICATION

(11) **EP 1 884 252 A1**
(43) Date of publication of application: **06.02.2008**
(21) Application number: 06118460.2
(22) Date of filing: 04.08.2006
(51) Int. Cl.: A61L 9/04, C08J 3/22

(54) **Polyolefin plastics with long-lasting odour**

(71) Applicant: Electrolux Home Products Corporation N.V., 1930 Zaventem (BE)
(72) Inventor: Breese, Kevin, 33080 Roveredo in Piano (Pordenone) (IT)
(74) Representative: Giugni, Valter

(57) **Abstract**

The present invention relates to a process for preparing odorant polyolefin plastics, to the odorant plastic itself, and also to a moulding composition and an article which comprises the odorant plastic.

In the process according to the invention a concentrate of an odour imparting material uniformly dispersed in a polyolefin or polyolefin copolymer material is mixed with a filler material and with a polyolefin material for forming a mixture that is processed to generate said odorant polyolefin plastics.

## Description

The present invention relates to a process for preparing odorant polyolefin plastics, to the odorant plastic itself, and also to a moulding composition and an article which comprises the odorant plastic.

In recent years odorant or fragrant plastics have become increasingly useful as a novel marketing tool for bringing products to the attention of the consumers. As is well known in the art, it is difficult to admix polyolefins with extraneous materials such as scent or aroma imparting material so as to obtain a product in which the scent or aroma imparting material is not only uniformly and homogeneously distributed, but also suitable for diffusing an odour for a relatively long period of time. In fact, if a scenting material in liquid form is added to raw polyolefin particles which, for manufacturing purposes, are heated to a liquid or highly viscous state in order to shape them into the desired form by moulding, casting, extrusion, etc., the scenting material has a tendency to separate and to form a separate phase.

So far, various attempts have been made to impart scents or odours to polyolefinic materials. A process known in the art consists in that plastic, which is generally in the shape of granules, is mixed with the required amount of scenting material (in a percentage of 1 to 10% by weight) and is subsequently processed. This process is rather uneconomical as the major part of the scenting material is evaporated already in the course of the processing or is heat degraded; in most cases the scenting material escapes from the processing machine without being combined with the polymer. The scenting material is deposited on the surface of the products because it is incompletely incorporated into the polymer matrix. The product is unsightly and blemished on the surface.

In a further known process for imparting odour to plastics, a polymer is mixed with scenting material at a ratio of 1:1.5 to 1:2 by weight and the mixture is processed in an extruding machine into granules containing up to 50% by weight of the scenting material. This product is usually called "masterbatch". The required amount of the masterbatch is thereafter added to the polymer prior to its processing into the final scented product. A drawback of this process is again the large loss and/or degradation of the expensive scenting material when the latter, together with the polymer, is exposed to high temperature for forming the final product.

It is an object of this invention to eliminate the drawbacks of known processes for preparing odorant polyolefin plastics and thus provide an improved process that allows preparation of polyolefin plastics with long-lasting odour.

Another object of the present invention is to provide an odorant plastic in which the scent emitting material is uniformly dispersed and slowly released to the environment.

Advantages, objects, and features of the invention will be set forth in part in the description which follows and in part will become apparent to those having ordinary skill in the art upon examination of the following or may be learned from practice of the invention. The objects and advantages of the invention may be realised and attained as particularly pointed out in the appended claims.

A process for preparing odorant polyolefin plastics according to the invention requires that a concentrate of an odour imparting material uniformly dispersed in a polyolefin or polyolefin copolymer is mixed with a filler material and with a proper amount of matrix polyolefin material so as to form a mixture that can be processed, for example, by pressing, injection moulding, extruding, blow moulding or the like to produce odorant polyolefin plastics.

Polyolefin polymers and copolymers in whose matrix a concentrate of an additive, like an odour emitting material, a pigment or a stabilizer, is uniformly dispersed are commonly known as "masterbatches". A masterbatch, normally in pellet form, is commonly used to achieve the proper concentration and dispersion of additives in plastic materials.

Masterbatches comprising scent emitting substances, like odorant essential oils, are commercially available from Add Master Limited and Polyvel Inc..

In order to produce odorant polyolefin plastics in which the odour is released for a relatively long time, a masterbatch of the type cited above, wherein the concentration of the odour imparting material in the polyolefin or polyolefin copolymer bulk is preferably 10 to 40% by weight, is mixed with a filler material. Such filler material is preferably selected from calcium carbonate compounds, like dolomite (MgCa(CO₃)₂) or calcium carbonate itself, silicate compounds, like mica (K₂Al₆Si₆O₂₀(OH)₄), talc (Mg₃Si₄O₁₀ (OH)₂) or silica itself. The filler material has been found to trap on its surface the odour molecules contained in the masterbatch and therefore to greatly slow odour release from the plastics obtained with the process described above.

A preferred amount of filler material to be added to the mixture of polyolefin material and masterbatch is comprised between 0.5 and 60% by weight. In the mixture, the masterbatch represents up to 10% by weight and the polyolefin material 30 to 99% by weight.

It has been found that 40% by weight of micron-sized calcium carbonate in polypropylene allows odorant plastics to retaining odour for at least three month at 40°C. The odorant plastic obtained with the process according to the invention may be in pellet form and may be used in combination with other additives and/or colorants for producing a moulding composition. It is envisioned that since the surface of the filler is holding the odour molecules higher surface area fillers, such as nanofillers, should have a higher efficacy for odour retention.

The odorant plastic and the odorant polyolefin plastic produced with the present process can be used for altering the odour properties of articles. In fact, incorporating such moulding composition or the polyolefin plastic in an article formed by a plastic moulding or a semi-finished product, any desired odour can be imparted to such article.

Particularly advantageous is the provision of odour articles in the form of component parts of a household appliance. In this case it is possible to impart an attractive fragrance to door plastic components of a washing and/or drying machine or to the inner cavity of a rotating laundry drum comprised in such machines using scented plastics for making the tumbling ribs provided in the inner side of said drum.

Conclusively it can be stated that the present invention allows the production of polyolefin plastics suitable for releasing a virtually unlimited variety of scents for a relatively long period of time. In addition, the present process for preparing odorant polyolefin plastics greatly reduces the loss of scenting material during ordinary articles forming operations.

## Claims

1. A process for preparing odorant polyolefin plastics in which a concentrate of an odour imparting material uniformly dispersed in a polyolefin or polyolefin copolymer material is mixed with a filler material and with a polyolefin material for forming a mixture that is processed to generate said odorant polyolefin plastics.

2. A process according to claim 1 in which the concentration of the odour imparting material in the first amount of polyolefin polymeric material is 10 to 40% by weight.

3. A process according to claim 1 or 2 in which the filler comprises a calcium carbonate compound.

4. A process according to claim 1 or 2 in which the filler comprises a silicate compound.

5. A process according to claim 3 or 4 in which the concentration of filler in the mixture is 0.5 to 60% by weight.

6. A process according to any preceding claim wherein said mixture is processed by pressing, injection moulding, extruding or blow moulding.

7. An odorant polyolefin plastic obtained by the process as claimed in any of claims 1 to 6.

8. An odorant plastic as claimed in claim 7 in pellet form.

9. A moulding composition comprising an odorant plastic as claimed in claim 7 or 8.

10. The use of the moulding composition or of the odorant plastic as claimed in claims 7 or 8 for altering the odour properties of articles.

11. An article comprising an odorant plastic as claimed in claim 7 or 8 and/or a moulding composition as claimed in claim 9.

12. An article as claimed in claim 11 in the form of a plastic moulding or a semi-finished product.

13. An article as claimed in claim 11 or 12 in the form of a component part of a household appliance.
